# EUROPEAN PATENT APPLICATION

(11) **EP 2 762 149 A2**
(43) Date of publication of application: **06.08.2014**
(21) Application number: 12834759.8
(22) Date of filing: 26.09.2012
(51) Int. Cl.: A61K 38/17, C07K 14/505, A61P 35/00

(54) **ERYTHROPOIETIN-DERIVED PEPTIDE AND USE THEREFOR**

(30) Priority: 27.09.2011 KR 20110097723
(71) Applicant: Kim, Hoojung, Seoul 150-796 (KR)
(72) Inventor: Kim, Hoojung, Seoul 150-796 (KR)
(74) Representative: Campbell, Patrick John Henry
(86) International application number: PCT/KR2012/007763
(87) International publication number: WO 2013/048116

(57) **Abstract**

Provided are a peptide, which has an amino acid sequence of SEQ ID NO. 1 and is effective for the prevention of cell damage, and a pharmaceutical composition for preventing cell damage, the pharmaceutical composition including the peptide as an active component. The peptide according to the present invention not only exhibits substantially better biological activities than conventional natural human erythropoietin in terms of various cell protection activities, but also has a substantially simpler structure than the natural human erythropoietin. Thus, the peptide may easily pass through a tissue-blood barrier and is economically advantageous due to its low production cost. Accordingly, the pharmaceutical composition including the peptide according to the present invention as an active component is effective for the prevention and treatment of stroke, mechanical damage or ischemic injury to the nervous system, myocardial infarction, diabetes, heart failure, peripheral vascular disorders, diabetic retinopathy, diabetic nephritis, diabetic neuropathy, glaucoma, neurodegenerative diseases such as Alzheimer's disease, Parkinson's disease, and Lou Gehrig's disease, and acute and chronic renal dysfunction.

## Description

### TECHNICAL FIELD

Provided are an erythropoietin-derived peptide and a pharmaceutical composition including the erythropoietin-derived peptide.

### BACKGROUND ART

During lifetime, a human body is consistently exposed to internal or external stimulations and hazards. In response to the hazards, an individual protects its body through various defense mechanisms. Such hazards include mechanochemical hazards, hypoxia, and infection. As such, many protection mechanisms against such hazards, more specifically, at a cellular level, are being discovered. Various cell factors that are secreted during the mechanisms protect the body from chemical and immunological damage caused by secondary internal hazards produced by apoptotic cells after the completion of protection mechanisms, in addition to initial external hazards. Thus, the cell factors also play a role in the prevention of apoptosis (or necrosis), prevention of expansion of an initial lesion, development of new cells, and induction of regeneration into new and healthy tissues.

Erythropoietin (EPO) is a well known hematopoietic cytokine composed of 165 amino acids and having a molecular weight of 34 kDa, which is produced in the kidneys in response to hypoxia in the body to stimulate precursors of red blood cells, to thereby increase the number of red blood cells.

Accordingly, recombinant human EPO (rhEPO) may be used as a medicine for treating anemia, such as anemia caused by chronic renal failure, anemia due to a surgery, anemia due to cancer or an anti-cancer treatment (non-patent document 1). Recently, EPO showed a cell and tissue protection capabilityin mechanical or ischemic injury to the nervous system (non-patent document 2), and showed a reduction of about 50% in tissue damage in animal models having acute myocardial infarction, and these effects are considered to via anti-apoptosis (non-patent documents 3 and 4).However, in addition to the treatment of anemia and the cell and tissue protection capability of the EPO, it has been found that blood circulatory disorder caused by increase of red blood cells and increase of platelet activity may occur due to the injection of EPO (non-patent documents 5 and 6). These adverse effects may lead to a decrease in the tissue protection capability of the EPO. Accordingly, research is being conducted into developing EPO derivatives, such as asialo EPO and carbamylated EPO, or peptides including a partial structure of EPO, which are capable of maintaining the tissue protection capability without increasing red blood cells or stimulating platelet activity (non-patent documents 7 to 9).

As described above, the EPO is known to play a very important role in the treatment of anemia by increasing red blood cells and in the protection of cells and tissues in the nervous system and the protection of myocardial damaged tissues. As such, EPO is a very active protein, but has a very high production cost, and when the EPO is injected into peripheral blood vessels, the EPO may not be transported to a target organ due to a tissue-blood barrier present in certain target organs, which causes difficulties in drug delivery (non-patent documents 10 to 12). Accordingly, an effective human EPO substitute having low cost of production and capable of being fluently transported to biological tissues is required.

### [Prior Art Documents]

### Non-patent documents:

1. Jelkmann, W. Biology of erythropoietin (1994) Clin. Invest. 72, S3-S10.
2. Brines, M. L. et al. Erythropoietin cresses the blood brain barrier to protect against experimental brain injury (2000) Proc. Natl. Acad. Sci. USA 97, 10526-10531.
3. Moon C. et al. Erythropoietin reduces myocardial infarction and left ventricular functional decline after coronary artery ligation in rats) (2003) Proc Natl Acad Sci U S A. 2003 September 30; 100(20): 11612-11617.
4. Siren et al. Erythropoietin prevents neuronal apoptosis after cerebral ischemia and metabolic stress (2001) Proc. Natl. Acad. Sci. USA 98, 4044-4049.
5. Wiessner, C. et al. Increased cerebral infarct volumes in polyglobulic mice overexpressing erythropoietin (2001) J. Cereb. Blood Flow Metab. 21, 857-864. pmid: 11435798.
6. Wolf, R. F., Erythropoietin administration increases production and reactivity of platelets in dogs (1997) Thromb. Haemostasis 78, 1505-1509. pmid: 9423803.
7. Imai, N. et al., Physicochemical and biological characterization of asialoerythropoietin. Suppressive effects of sialic acid in the expression of biological activity of human erythropoietin in vitro) (1990) Eur. J. Biochem. 194, 457-462.
8. Leist M et al., Derivatives of erythropoietin that are tissue protective but not erythropoietic. Science (New York, NY 2004;305:239-42.
9. Brines M, et al., Nonerythropoietic, tissue-protective peptides derived from the tertiary structure of erythropoietin. Proc Natl Acad Sci USA. 2008; 105:10925-30.
10. Marti et al., Detection of erythropoietin in human liquor:intrinsic erythropoietin production in the brain, 1997, Kidney Int. 51:416-8.
11. Juul et al., Erythropoietin in the cerebrospinal fluid of neonates who sustained CNS injury, 1999, Pediatr, Res. 46:543-547.
12. Buemi et al. Intravenous recombinant erythropoietin does not lead to increase in cerebrospinal fluid reythropoietin concentration, 2000, Nephrol. Dial. Transplant. 15:422-433.2.

### DETAILED DESCRIPTION OF THE INVENTION

### TECHNICAL PROBLEM

To prepare a peptide having the same effects as natural human erythropoietin (EPO), a lower production cost than the natural human EPO, and a capability to pass through a tissue-blood barrier in the body, the inventors of the present invention prepared and screened various types of human EPO derived peptides. As a result, a peptide having substantially better biological activity than the natural human EPO, a capability to easily pass through the tissue-blood barrier, and a low production cost was selected from among many peptides to complete the present invention.

Accordingly, the objective of the present invention is to provide a peptide having better biological activity than the natural human EPO, a capability to pass through the tissue-blood barrier, and a low production cost.

Another objective of the present invention is to provide a pharmaceutical composition for preventing cell damage, the pharmaceutical composition including the peptide.

### TECHNICAL SOLUTION

According to an aspect of the present invention, there is provided a peptide effective for preventing cell damage, the peptide having an amino acid sequence of SEQ ID NO. 1.

According to another aspect of the present invention, there is provided a pharmaceutical composition for preventing cell damage including the peptide, which is effective for preventing cell damage, as an active component.

Hereinafter, embodiments of the present invention are described in greater detail.

All technical terms as used herein have the same meaning as those generally understood by one of ordinary skill in the art. Also, preferred methods or samples are described in the present specification, but similar or same methods or samples are incorporated herein. All publications described as cited documents in the present specification are incorporated herein in their entireties by reference.

The inventors of the present invention prepared and screened various types of human erythropoietin (EPO) derived peptides and found a peptide having better biological activities than the natural human EPO. The human EPO derived peptide is capable of passing through a tissue-blood barrier due to a substantially simpler structure than the natural human EPO and are economical due to their low production cost.

Accordingly, according to an aspect of the present invention, provided is a peptide having an amino acid sequence of SEQ ID NO. 1, which is represented by General Formula I, and is effective for prevention of cell damage.

[General Formula I]
Ile Ser Gly Leu Arg Ser Leu Thr Thr Leu Leu Arg Ala Leu Gly Ala Gln Lys Glu Leu
Met

General Formula I, which represents the peptide according to the present invention, is described above to conveniently express the structure of the peptide of the present invention. It should be understood by one of ordinary skill in the art that modified sequences of the peptide are also included in the present invention.

According to an embodiment of the present invention, a protecting group selected from the group consisting of an acetyl group, a fluorenyl methoxycarbonyl group, a formyl group, a palmitoyl group, a myristyl group, a stearyl group, and polyethylene glycol (PEG), and amino acids, may be additionally bound to an N-terminal or a C-terminal of the peptide of the present invention.

According to another embodiment of the present invention, an N-terminal or a C-terminal of the polypeptide of the present invention may be modified to a hydroxy group (-OH) or an amino group (-NH₂), and more preferably to a hydroxy group.

The modification of amino acids may substantially improve stability of the peptide of the present invention. The term "stability" as used herein refers to *in* vivo stability and storage stability (for example, room temperature storage stability). The protecting group may protect the peptide of the present invention from proteases *in vivo.*

The term "peptide" as used herein may be a linear molecule formed by binding amino acids through peptide bonds. The peptide of the present invention may be prepared according to a synthesis method known in the art, for example, a solid-phase synthesis technique (Merrifield, J. Amer. Chem. soc. 85:2149-54(1963); Stewart et al., Solid Phase Peptide Synthesis, 2nd, ed., Pierce Chem. Co.: Rockford, 111(1984)).

The peptide may be derived from human EPO and the peptide itself is known to have substantially better nerve cell protection, cardiomyocyte protection, retinocyte protection, and pancreatic β-cell protection than the natural human EPO (Experimental Examples 1-4).

According to another aspect of the present invention, provided is a pharmaceutical composition including the peptide according to the present invention for prevention or treatment of damage in cells, tissues, or organs.

Also, provided is a pharmaceutical composition including a pharmaceutically effective amount of the peptide according to the present invention and a pharmaceutically acceptable carrier, for treatment of damage in cells, tissues, or organs.

As used herein, the term "damage" refers to any human diseases or states, in which the peptide according to the present invention has prevention or treatment effects on neurological or psychiatric symptoms, eye diseases, cardiovascular diseases, blood pressure diseases, respiratory diseases, kidney, urinary tract, and genital diseases, bone diseases, skin diseases, gastrointestinal diseases, and endocrine and metabolic disorders. More specifically, such diseases or states include a hypoxia state, which negatively affects excitable tissues, such as excitable tissues of the central nervous system, peripheral nervous system, and cardiomyocytes or renal tissues, for example, brain/spinal cord, heart, or retina/eye.

A decrease in a possibility of using oxygen in a nervous tissue may lead to stress, damage and ultimately, apoptosis of neurocytes, which may be treated by the peptide according to the present invention.

Generally, states commonly referred to as hypoxia and/or ischemia include stroke, vascular congestion, lack of oxygen before or after birth, asphyxia, airway obstruction, asthma, drowning, carbon monoxide poisoning, smoke inhalation, trauma including surgery and radiation therapy, epilepsy, hypoglycemia, chronic pulmonary circulation disorder, emphysema, adult respiratory distress syndrome, hypotension shock, septic shock, anaphylactic shock, insulin shock, sickle erythrocyte seizure, arrhythmia, nitrogen anesthesia, and neurologic deficits caused by a heart-lung transplant, but it is not limited thereto. As such, the peptide according to the present invention may be used to treat or prevent deficiency of excitable tissues caused by hypoxia in various states and environments. Examples of the disease or the state include ischemia (coronary artery disease, myocardial infarction, angina pectoris, valvular cardiomyopathy as a congenital heart disease, Prinz-metal angina pectoris, cardiac rupture, vasculitis, arrhythmia, myocarditis, blunt trauma, penetrating trauma, toxins such as cocaine), high blood pressure, decompression sickness, muscle fiber hyperplasia syndrome, aneurysm, asthma , chronic bronchitis, emphysema, pulmonary embolism, pulmonary thrombosis, idiopathic pulmonary fibrosis, cystic fibrosis, pneumonia, sarcoid syndrome, type 1 or type 2 diabetes mellitus, osteopenia, osteomyelitis, avascular necrosis, trauma, Paget's disease, alopecia, vitiligo, diabetic ulcers, burns, peripheral vascular disease, lupus erythematosus, Sjögren's syndrome, rheumatoid arthritis, glomerulonephritis, vasculitis, visual neuritis, retinal detachment, temporal arteritis, retinal ischemia, macular degeneration, retinitis pigmentosa, camellia cure, retinopathy, hypertensive retinopathy, retinal artery occlusion, retinal vein occlusion, diabetic retinopathy, suffocation, ischemia, eclampsia, ischemic stroke, hemorrhagic stroke, brain trauma, spinal cord trauma, epilepsy, seizure, chronic seizure disorder, chronic fatigue syndrome, and acute and chronic hyperosmotic hypotonic syndrome, AIDS, dementia, electrocution, suffocation, multiple sclerosis, Alzheimer's disease, cerebral palsy, aging of brain function, memory loss, ALS, seizure disorder, subacute sclerosing encephalitis, encephalitis, meningitis, subdural hematoma, nicotine addiction, drug abuse and withdrawal, obsessive mental disorders, mood disorders, anxiety disorders, depression, autism, attention deficit / hyperactivity disorder, cognitive dysfunction, spinal stenosis, crossing spondylitis, Gillian-Barre syndrome, trauma, neuromuscular compression, oncothlipsis, heat cerebral infarction, tuberous sclerosis, Wilson's disease, cerebral and progressive supranuclear palsy, Guam disease, Lewy body dementia, Huntington's disease, myotonic dystrophy, Friedrich ataxia and other ataxia, Tourette's syndrome, Creutzfeldt Jakob disease (CJD), tinnitus, Meniere's syndrome, hearing loss, traumatic injury, diabetic kidney, Henoch-Schonlein purpura, myasthenia gravis, dermatomyositis, polymyositis, myopathy, rhabdomyolysis, mitochondrial disease, necrotizing fasciitis, secondary impotence caused by drugs, viral/ bacterial / parasitic hepatitis, cirrhosis, fatty liver, infiltration / metabolic disease of the liver, ischemic bowel disease, inflammatory bowel disease, necrotizing gastroenteritis, state of a donor and a recipient of an organ transplant, infertility (vascular, autoimmunity, uterine disorders, and implantation failure), endocrine hyperactivity, or endocrine dysfunction, but it is not limited thereto.

The pharmaceutical composition according to the present invention includes the peptide according to the present invention as an active component, wherein the peptide protects cardiomyocytes, neurocytes, retinocytes, and pancreatic β-cells and thus, the pharmaceutical composition may be effective for preventing or treating damage in cells, tissues, and organs by preventing a cell damage. Also, due to such effects, the pharmaceutical composition may be used for prevention or treatment of various diseases listed above including stroke, mechanical or ischemic damage in the nervous system, myocardial infarction, retinal damage, and diabetes, which may cause damage to cells, tissues, or organs. Preferably, the pharmaceutical composition may be used to protect from or repair functional damage related to cerebral infarction disability, multiple sclerosis, attack, low blood pressure, cardioplegia, ischemia, myocardial infarction, inflammation, age-related loss of cognitive function, radiation damage, cerebral palsy, neurodegenerative diseases, Alzheimer's disease, Parkinson's disease, Leigh's disease, AIDS dementia, memory impairment, amyotrophic lateral sclerosis, alcoholism, mood disorder, anxiety disorder, attention deficit disorder, autism, Creutzfeldt-Jakob disease, brain or spinal cord injury or ischemia, heart-brain bypass, chronic heart failure, decreased vision due to aging, diabetes, diabetic neuropathy, diabetic kidney failure, glaucoma, retinal ischemia, or retinal trauma.

As used herein, the expression "pharmaceutically effective amount" refers to an amount sufficient to achieve effects of the peptide *in vivo.*

The pharmaceutical composition may be prepared as a general pharmaceutical formulation known in the art. The pharmaceutical composition may be administered orally or parenterally, or more preferably, parenterally and when the pharmaceutical composition is administered parenterally, the pharmaceutical composition may be administered through intravenous infusion, subcutaneous injection, intramuscular injection, intraperitoneal injection, topical administration, or transdermal administration. Accordingly, the pharmaceutical composition may be a formulation for oral administration, an injection, a suppository, a transdermal formulation, and a nasal formulation, but the pharmaceutical composition is not limited thereto and may be prepared as any formulation.

When the pharmaceutical composition is prepared as the formulation described above, a pharmaceutically acceptable carrier or an additive needed for the preparation of each formulation may be added. When the pharmaceutical composition is formulated as an injection, the injection may be prepared according to a general method of preparing an injection. According to an embodiment, the injection according to the present invention may be prepared by dissolving a drug in general distilled water. The injection according to the present invention may in the form of a medicament dispersed in a sterilized medium, such that the injection may be used as it is, or may be in the form of a medicament which is dispersed at suitable concentration through the addition of distilled water for injection just before the administration thereof.

When the pharmaceutical composition is formulated into a formulation for oral administration, at least one selected from a diluent, a lubricant, a binder, a disintegrating agent, a sweetening agent, a stabilizer, and a preservative may be used as a carrier and at least one selected from a spice, a vitamin, and an antioxidant may be used as an additive. Any pharmaceutically acceptable carrier and additive may be used, preferably, lactose, cornstarch, soybean oil, microcrystalline cellulose, or mannitol may be used as the diluent; magnesium stearate or talc as a lubricant agent; and polyvinylpyrrolidone or hydroxypropyl cellulose as a binder. Also, the disintegrating agent may preferably be carboxymethylcellulose calcium, sodium starch glycolate, polacrilin potassium, or crospovidone and the sweetening agent may preferably be sucrose, fructose, sorbitol, or aspartame and the stabilizer may preferably be sodium carboxymethylcellulose, beta-cyclodextrin, bleached beeswax, or xanthan gum and the preservative may preferably be methyl p-oxybenzoate, propyl p-oxybenzoate, or potassium sorbate.

A dose of the pharmaceutical composition according to the present invention may be determined by various factors, such as a method of formulation, a mode of administration, age, weight, sex, and morbidity of a patient, food, administration time, administration route, excretion rate, and reaction sensitivity, and the peptide may be administered in a range of about 0.001 *µg*/day to about 100 *µg*/day, based on a standard adult male.

### ADVANTAGEOUS EFFECTS

As described above, the peptide according to the present invention has substantially better biological activity than conventional natural human erythropoietin (EPO) with respect to protection of various cells as well as a substantially simpler structure than the natural human EPO, such that the peptide may easily pass through a tissue-blood barrier and has a low production cost. Accordingly, a pharmaceutical composition including the peptide as an active component may be effective for prevention or treatment of various diseases related to cell damage such as stroke, mechanical damage or ischemic damage to the nervous system, myocardial infarction, retinal damage, and diabetes.

### DESCRIPTION OF THE DRAWINGS

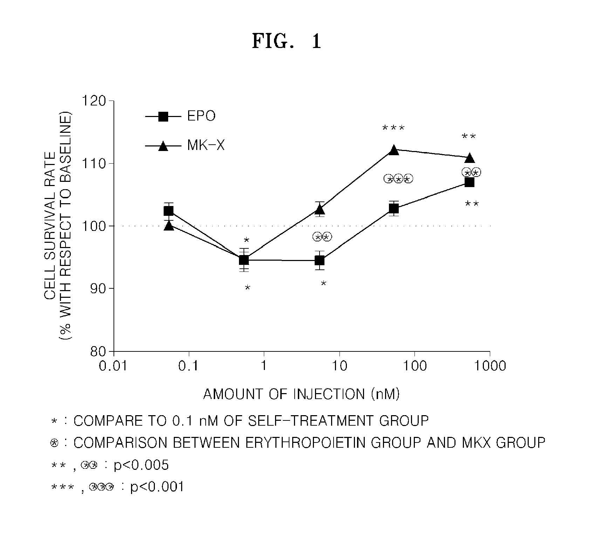

FIG. 1 is a graph showing results of comparing cell survival rates after treating cardiomyocytes with erythropoietin (EPO) and MKX-2 at various concentrations, culturing the cardiomyocytes for 72 hours, and measuring the cell survival rates through an MTT assay.
   In an EPO graph, a point located at the bottom portion of the graph lacks an asterisk '*' because there was no statistically significant difference in cell survival rate compared to the cell survival rate at a reference concentration of 0.1 nM. Among the three double asterisks '**' at a concentration of 1000 nM, the upper ** indicates that there was a statistically significant difference for 1000 nM of MKX-2 compared to 0.1 nM of MKX-2, the middle 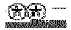 indicates that there was a statistically significant difference in cell protection between 1000 nM of MKX-2 and 1000 nM of EPO, and the bottom ** indicates that 1000 nM of EPO had a statistically significant difference compared to 0.1 nM of EPO.
FIG. 2 is a graph showing results of comparing cell survival rates after treating brain cells with EPO and MKX-2 at various concentrations, culturing the brain cells for 72 hours, and measuring the cell survival rates through an MTT assay.
FIG. 3 is a graph showing results of comparing cell survival rates after treating retinal neurocytes with EPO and MKX-2 at various concentrations, culturing the retinal neurocytes for 72 hours, and measuring the cell survival rates through an MTT assay.
FIG. 4A is a graph showing results of comparing cell survival rates after pre-treating HIT-T15 β-cells with MKX-2 at various concentrations, treating the β-cells with H₂O₂ for 2 hours, and measuring the cell survival rates through an MTT assay.
FIG. 4B is a graph showing results of comparing cell survival rates after pre-treating HIT-T15 β-cells with MKX-2 at various concentrations, treating the β-cells with H₂O₂ for 4 hours, and measuring the cell survival rates through an MTT assay.
FIG. 5A is a graph showing results of comparing cell survival rates after pre-treating INS-1 β-cells with MKX-2 at various concentrations, treating the β-cells with H₂O₂ for 2 hours, and measuring the cell survival rates through an MTT assay.
FIG. 5B is a graph showing results of comparing cell survival rates after pre-treating INS-1 β-cells and MKX-2 at various concentrations, treating the β-cells with H₂O₂ for 4 hours, and measuring the cell survival rates through an MTT assay.
FIG. 6 shows images showing frontal slices of brains which were separated from cerebral infarction induced Sprague-Dawley rat models that were fed with a test drug and then dyed with TTC.
FIG. 7 is a graph showing measurement results of cerebral infarction area of a frontal slice of brain which was separated from a cerebral infarction induced Sprague-Dawley rat model that was fed with a test drug and then dyed with TTC.
FIG. 8 is a graph showing calculation results of a ratio of the number of DAPI dyed cells to TUNEL positive cells in the results of TUNEL assay with respect to cultured cardiomyocytes, which were obtained from a Wister rat.

### BEST MODE

Hereinafter, the present invention will be described in greater detail through the embodiments described below. However, the embodiments are for illustrative purposes only and do not limit the scope of the invention.

### <Example>

### 1. Synthesis of Ile Ser Gly Leu Arg Ser Leu Thr Thr Leu Leu Arg Ala Leu Gly Ala Gln Lys Glu Leu Met

A peptide sequence of Ile Ser Gly Leu Arg Ser Leu Thr Thr Leu Leu Arg Ala Leu Gly Ala Gln Lys Glu Leu Met, which is a SEQ ID NO.1, was synthesized according to a solid-phase synthesis technique known in the art.

The peptide was named MKX-2.

### <Experimental Example>

### Method and materials for the experiments

### 1. Method and materials for a myocardiocyte experiment

### A. Separation and culturing of cardiomyocytes

An 8 weeks old Wister rat was injected with ketamine and pentobarbital to quickly kill the mouse, and the heart was quickly separated from the mouse. An aorta was used to perfuse with Ca free Krebs-Henseleit buffer (KHB) solution, which was pre-heated to a temperature of about 34°C to about 37°C, in heart direction. Thereafter, the KHB solution was switched to Hank's balanced salt solution (HBSS:GIBCO, Grand Island, NY) containing 0.15% class II collagenase (Cooper Biomedical, Malvern, PA) and then perfused for about 30 to about 40 minutes. Thereafter, the product obtained therefrom was washed twice with a cell culture medium (Dulbecco's modified Eagle's medium, DMEM). Ventricles and atria were separated therefrom, ventricular tissues were thoroughly chopped and then stirred thoroughly for about 5 minutes in a shaker filled with cell culture medium (DMEM) containing class II collagenase that was pre-heated to a temperature of about 34°C to about 37°C. Connective tissues that were not dissolved were filtered and a collagenase-containing solution was washed twice with the cell culture medium (DMEM). The product obtained therefrom was gently centrifuged at a speed of 10 g for 2 minutes, a supernatant obtained therefrom was separated and removed, and cell components were washed three times with a cell culture medium (DMEM) solution containing 1% fetal bovine serum (FBS; Sigma Chemical, St. Louis, MO). Thereafter, the cells obtained therefrom were stabilized in a cell culture medium (DMEM), 10% FBS, 10mM N-2-hydroxyethylpiperazine-N'-2-ethanesulfonic acid (HEPES, GIBCO Laboratories, Grand Island, NY), 2mM L-glutamine, penicilin-stereptomycin (100 IU/mL, GIBCO) for 60 minutes.

Thereafter, the cells were divided in a 6-well plate at a density of 20X10³ cells/cm² and then bromodeoxyuridine (BrdU, 10uM, Sigma-Aldrich) was added to the cell culture medium over 3 days to prevent the proliferation of fibroblasts and progenitor cells (Lokuta et al, 1994; Miragoli et al., 2006). The cells were cultured in an incubator under conditions of 95% of O₂, 5% of CO₂, and a temperature of 37°C for the next experiment.

### B. MTT assay

Cells were seeded in a 96-well plate at a density of 2x10⁴ cells/well and 200 ul for each well. In this regard, cell confluence was set at about 70% to about 80%. After 24 hours, when the cells have been stabilized, a 26 gauge or 28 gauge needle was used to remove a preexisting medium from the wells. 200 ul of PBS free of serum and nutrients was added to each well, the wells were divided into erythropoietin (EPO) treatment groups and MKX-2 treatment groups, which were further divided into experimental groups according to concentrations of 0 nM, 0.1 nM, 1 nM, 10 nM, 100 nM, and 1000 nM and then treated with a drug for 72 hours in a hypoxic condition, and then the cells were subjected to an MTT assay to compare cell survival rates. The EPO used was an Eporon injection (Dong-A Pharmaceuticals, Korea). All experimental groups for each concentration included 7 wells.

After a predetermined treatment time, 3-[4,5-dimethylthiazol-2-yl]-2,5-diphenyl tetrazolium bromide (MTT, Sigma, M2128) that was dissolved in a phosphate buffered saline (PBS) and then sterilization filtered by using a membrane filter having a thickness of 0.22 um was added to each well in an amount of 100 ul at a concentration of 1 mg/ml. In this regard, an MTT reagent was used after wrapping the MTT reagent with a foil sheet to block from light. The well plate added with the MTT reagent was reacted in an incubator for 2 hours. A medium was removed after culturing. While removing the medium, care was taken to prevent cells from being removed with the medium.

200 ul of DMSO was added to each well. The well plate were wrapped with a foil sheet and then gently stirred by using a shaker for 10 minutes to thoroughly dissolve formazan crystals. A multi-pipette (160ul) was used to break the formazan crystals through pipetting. An ELISA reader was used to measure an O.D. value at 570 nm for quantitative analysis.

### 2. Method and materials for neurocyte experiment

### A. Separation and culturing neurocytes

### (1) Plate coating

Poly-L-lysine (sigma P4707) was added to a plate for culturing cells and then maintained in an incubator for 1 hour. The poly-L-lysine was suctioned by using a pipette and then the plate was washed three times with sterilized distilled water. Coated plate was completely dried in a clean bench for 2 hours or more, wrapped with a plastic film, and then refrigerated.

### (2) Preparation of a medium

### 1) Fetus washing medium or HBSS (200 ml/fetus)

HBSS(GIBCO, 14170-112, 180ml) and 10% penicilin/streptomycin (GIBCO, 15140-122, 20ml) were mixed to be prepared.

### 2) Trypsin solution

0.125% trypsin-EDTA (3ml), HBSS (12ml), 0.1 mg/ml DNase1 (sigma, deoxyribonuclease1, D5025, 1.5mg) were mixed and then filtered by using a syringe.

### 3) Basal media (1 L in total)

A bag of DMEM (hg, GIBCO, 12100-046), 1% P/S 10 ml(penicillin/streptomycin, GIBCO, 15140-122), and 3.7g of NaHCO₃ (sodium bicarbonate, sigma, S5761) were mixed with 0.8 L of distilled water and then filtered by using a syringe.

### 4) Culturing media (1 L in total)

900 mL of basal media, 100 mL of 10% FBS, 500 ul of insulin (sigma, I9278-5ml, 10mg/ml), and 100 ul of 70 mM p-aminobenzoate (PABA : N-acetyl transferase inhibitor, A9878) were mixed and then filtered by using a syringe.

### 5) Washing media (20ml/fetus)

A bag of DMEM (hg, GIBCO, 12100-046), 20ml of 1% P/S, and 2 mg of 0.1 mg/ml DNase1 were mixed and then filtered by using a syringe.

### (3) Separation and culturing of neurocytes

A rat pregnant for 16 weeks was cervically dislocated to sacrifice the experimental animal and the abdomen of the experimental animal was excised in an U-shape to expose the uterus in which a fetus was located. Thereafter, the fetus was taken out from the uterus and then inserted into a fetus washing medium and then washed three times. Only the head of the fetus was cut and then inserted into the fetus washing medium to wash the head three times and then the cranium of the fetus was broken to separate the brain therefrom. A knife was used to cut the cortex of the brain and tissues of the cortex were thoroughly chopped, which were then collected in the already prepared fetus washing medium. After centrifuging at 400 g for 4 minutes, a supernatant was removed therefrom and then 0.025% of trypsin and 10 ml of DNase1 were added to the pellets to break the pellets. The mixture was stored in an incubator for 15 minutes.

Thereafter, 15 ml of a cell culture medium was added thereto again, centrifuged at 400 g for 4 minutes, a supernatant was removed therefrom to obtain pellets, which were washed twice with 6 ml of a washing medium (including DNase 1), and then centrifuged again at 400 g for 4 minutes. After the centrifugation, the supernatant solution was removed again to obtain pellets, 10 ml of the culture medium was added to the pellets and a mixture obtained therefrom was mixed with 10 ul of trypan blue to count the cells by using a hemocytometer. In a 24-well plate, 500 ul of the cells were seeded in each well and then cultured for 48 hours. Thereafter, the culture medium was removed from the well plate for stabilization, a new cell culture medium and 5 uM Ara-c (cytosine arabinoside, sigma, C1768) were added thereto, cultured for 14 hours, the culture medium was removed again after 24 hours, a new culture medium was added thereto, and then cultured in an incubator having conditions of 95% O₂, 5% CO₂, and a temperature of 37°C for the next experiment.

### B. MTT Assay

In a 96-well plate, cells were inoculated at a density of 2x10⁴ cells/well and in an amount of 200 ul for each well. In this regard, cell confluence was set to be about 70% to about 80%. After 24 hours, when the cells have stabilized, a 26 or 28 gauge needle was used to remove a pre-existing medium from the wells. 200 ul of PBS free of serum and nutrients was added to the wells and the wells were divided into an EPO treatment group and an MKX-2 treatment group, and the wells in the EPO treatment group were starved of serum to be further divided into three groups, namely, an EPO non-treatment group, a 1 IU/ml treatment group, and a 10 IU/ml treatment group, and each group was further divided into a treatment group which was treated with 0.1 % 2-mercaptoethanol, which is a neurotoxin, for 18 hours, and a non-treatment group to compare different groups.

Similarly, the MKX-2 treatment group was removed of the serum, divided into three groups of an MKX-2 non-treatment group, a treatment group treated with 1.2 ng/ml of MKX-2, and a treatment group treated with 1.2 ng/ml of MKX-2, each of which was divided into a non-treatment group and a treatment group that was treated with 0.1% 2-mercaptoethanol, which is a neurotoxin, for 18 hours.

Also, as a negative control group, a group cultured in normal culture medium conditions was prepared for comparison. In each experimental condition, a total of 6 wells were tested and average and standard deviation were used in statistical analyses. The EPO and MKX-2 treatment groups were cultured in drug treatment conditions for 72 hours each. The EPO was an Eporon injection (Dong-A Pharmaceuticals, Korea). After a certain treatment time, 3-[4,5-dimethylthiazol-2-yl]-2,5-diphenyl tetrazolium bromide (MTT, Sigma, M2128) that was dissolved in a phosphate buffered saline (PBS) and then sterilization filtered by using a membrane filter having a thickness of 0.22 um was added to each well in an amount of 100 ul at a concentration of 1 mg/ml. In this regard, an MTT reagent was wrapped with a foil sheet to block it from light. The well plate, in which the MTT reagent was added, was reacted for 2 hours in an incubator. A culture medium was removed after culturing. When removing the medium, care was taken to prevent cells from being removed with the medium. Thereafter, 200 ul of DMSO was added to each well. The wells were wrapped with a foil sheet and then gently stirred by using a shaker for 10 minutes to thoroughly dissolve formazan crystals. A multi-pipette (160ul) was used to break the formazan crystals through pipetting. An ELISA reader was used to measure an O.D. value at 570 nm for quantitative analysis.

### 3. Method and materials for retinal neurocytes experiment

### A. Separation and culturing of retinal neurocytes

### (1) Plate coating

Poly-D-lysine (sigma P7280) was added to a plate for culturing cells and then incubated in an incubator for 2 hours. Poly-D-lysine was suctioned out by using a pipette and then washed three times with sterilized distilled water. A coated plate was completely dried in a clean bench for 2 hours or more and then wrapped with a plastic film to be stored in a freezer.

### (2) Preparation of a culture medium

### 1) Solution 1

A medium having the following composition (5.4 mM KCI, 116 mM NaCl, 0.096 mM NaH₂PO₄2H₂O, 19.5 mM D-glucose, 0.15 mM MgSO4, 23.8 mM NaHCO₃, 3 g/L bovine serum albumin, and 10 mg/L phenol-red) and 20 ml of 10% penicillin/streptomycin (GIBCO, 15140-122) were mixed.

### 2) Trypsin solution

0.125% trypsin-EDTA (3ml), solution 1 (12 ml), and 1.5mg of 0.1 mg/ml DNase1 (sigma, deoxyribonuclease1, D5025) were mixed and then filtered through a syringe.

### 3) A stop solution of solution 1

DNAse (10000U/7.5 ml bovine pancrease Type II), soybean trypsin inhibitor (type 1-S, 5mg/7.5 ml), and 0.19 mM MgSO₄ were added to solution 1 to prepare a stop solution.

### 4) Minimum essential medium (1 L in total)

900 mL of basal media, 100 mL of 10% FBS, 91 mg/L gentamicin sulfate, 2.3 mg/L amphotericin B, and 5 mM glucose were mixed and then filtered by using a syringe.

### (3) Separation and culturing of renal neurocytes

Eyes of a 3 year old rat were removed and retina of the eyes were separated and then put into solution 1. Retinal tissues were treated in trypsin at a temperature of 37°C for 10 minutes and then centrifuged at 180g for 5 minutes.

After centrifugation, a supernatant solution was discarded and all collected tissues were put into a stop solution. Aggregated tissues were disentangled by using a pipette and then centrifuged again at 180 g for 5 minutes. Separated cells were mixed with 10% FBS-MEM at a density of 1X10⁶ cells/mL. 100 ul of cells were inoculated in each well of a 96-well plate and then cultured for 24 hours.

Thereafter, the culture medium was removed from the well plate for stabilization, a new culture medium and 5 uM Ara-c (cytosine arabinoside, sigma, C1768) were added thereto, cultured for 48 hours, the culture medium was removed again after 48 hours, a new culture medium was added thereto, and then cultured in an incubator having conditions of 95% O₂, 5% CO₂, and a temperature of 37°C for the next experiment. Thereafter, the culture medium was removed for stabilization after another 48 hours, then a new culture medium was added thereto, and then cultured in an incubator under conditions of 95% 02, 5% CO2, and a temperature of 37 °C for the next experiment.

### B. MTT assay

Cells were seeded in a 96-well plate at a density of 2x10⁴ cells/well and 200 ul for each well. In this regard, cell confluence was set to be about 70% to about 80%. After 24 hours, when the cells have stabilized, a 26 gauge or 28 gauge needle was used to remove a preexisting medium from the wells. 200 ul of PBS free of serum and nutrients was added to each well, the wells were divided into EPO treatment groups and MKX-2 treatment groups, which were further divided into experimental groups according to concentrations of 0 nM, 0.1 nM, 1 nM, 10 nM, 100 nM, and 1000 nM and then treated with a drug for 72 hours under hypoxic conditions, and surviving renal neurocytes were quantitatively analyzed in an MTT assay including a fluorescent material and a fluorescence analyzer. The EPO used was an Eporon injection (Dong-A Pharmaceuticals, Korea). All experimental groups for each concentration included 7 wells. After a certain treatment time, 3-[4,5-dimethylthiazol-2-yl]-2,5-diphenyl tetrazolium bromide (MTT, Sigma, M2128) that was dissolved in a phosphate buffered saline (PBS) and then sterilization filtered by using a membrane filter having a thickness of 0.22 um was added to each well in an amount of 100 ul at a concentration of 1 mg/ml. In this regard, an MTT reagent was used after wrapping the MTT reagent with a foil sheet to block it from light. The well plate that had the MTT reagent added thereto was reacted in an incubator for 2 hours.

A medium was removed after culturing. While removing the medium, care was taken to prevent cells from being removed with the medium. Thereafter, 200 ul of DMSO was added to each well. The wells were wrapped with a foil sheet and then gently stirred with a shaker for 10 minutes to thoroughly dissolve formazan crystals. A multi-pipette (160ul) was used to break the formazan crystals through pipetting.

Thereafter, an ELISA reader was used to measure an O.D. value at 570 nm for quantitatively analysis.

### 4. Method and materials for pancreatic β-cell experiment

### A. Culturing pancreatic islet cells

HIT-T15 cells (75 to 77 passages), which are from an insulin secretion cell line derived from a hamster, and INS-1 cells (18 to 20 passages), which are from an insulin secretion cell line derived from a rat, were used. A culture medium used for culturing HIT-T15 cells was 10% FBS, 100 units/ml of penicillin, and 100 g/ml of streptomycin added to RPMI-1640. The culture medium used for culturing INS-1 cells was 10% FBS and 1 mM pyruvate, 10 mM HEPES, 50 mM 2-mercaptoethanol, 100 units/ml penicillin, 100 g/ml of streptomycin mixed in RPMI-1640. Two β-cell lines were cultured in conditions in which 95% humidity was maintained at a temperature of 37°C and included 5% CO2. When cell confluence reached about 70% to about 80%, the cells were washed with PBS and then treated with 0.05% trypsin-EDTA to subculture the cells.

### B. MTT assay

β-cells were divided into wells in a 24-well plate at a concentration of 1 x 10⁵ cells/well and the β-cells were cultured for 24 hours such that a cell confluence reached about 70% to about 80%. After 24 hours, a pre-existing medium was removed and then replaced with RPMI-1640 including 0.5% FBS, cultured for 6 hours, and then the β-cells were inhibited from proliferation and differentiation. MKX-2 was dissolved in DMSO and then was used to pre-treat the β-cells at concentrations of 0 ng/ml, 0.12 ng/ml, 1.2 ng/ml, 12 ng/ml, 60 ng/ml, and 120 ng/ml, 1 hour prior to a H₂O₂ treatment. A control group was treated with DMSO at 0.001 % to be compared to the treatment group. 1 hour after an MKX-2 treatment, a H₂O₂ treatment was performed for about 2 hours or about 4 hours. A MTT (5 mg/ml) solution dissolved in PBS was added to each well and reacted for about 2 to about 3 hours, a supernatant was removed therefrom, such that formazanes formed at the bottom of the wells did not disperse, 250 ul of DMSO was added thereto and dissolved, and then an ELISA reader (Molecular Devices Exax, Sunnyvale, CA) was used to measure absorbance at 540 nm (ref. 650 nm). An absorbance value was quantitatively analyzed to calculate a cell growth inhibition rate

### 5. Method and materials for cerebral infarction animal experiment

### A. Experimental animal and cerebral infarction experiment model

In the present experiment, Spague-Dawley (SD) rats weighing about 280 g to about 330 g were used, which were purchased from Koatech Inc. (Seoul, Korea). The number of rats used was 39, wherein 13 of them were used as a control group and another 26 were divided into 13 in a MKX-2 treatment group and 13 in an EPO treatment group.

To induce cerebral infarction, a method of temporarily blocking a left middle cerebral artery and then reperfusing the same was used, and an intraluminal filament technique, which was modified from the Koizumi method was used. Then, each SD rat was anesthetized with a mixture gas of 70% nitrous oxide and 30% oxygen along with isoflurane (3% for induction and 2% for surgical procedure). During the surgery, an excision was made slightly leftwards from the center of the neck and the left common carotid artery was carefully separated without damaging surrounding vagus nerves.

The left common carotid artery was pulled by using black silk (3.0) and the ipsilateral internal carotid artery and the external carotid artery were separated, wherein the ipsilateral internal carotid artery was ligated first and then the external carotid artery was pulled thereafter. A 4-0 nylon thread (Ethilon; Ethicon Norderstedt, Germany) having a length of about 20 nm that was pre-coated with silicon resin (Xantopren; Bayer Dental, Osaka, Japan) was pushed into a hole made in the external carotid artery to block an origin of the left common carotid artery. After insertion, the external carotid artery and residual nylon thread were ligated to prevent bleeding or pushing out of the nylon thread.

The surgery was performed without bleeding for about 15 minutes. After two hours, the nylon thread was removed, such that a complete reperfusion through the left common carotid artery could occur.

Right after the blockage/reperfusion of the middle cerebral artery, a 0.9% saline solution, 3.6 µg/kg MKX-2, or 3000 iu/kg erythropoietin (eporon, Dong-A Pharmaceuticals) was injected to each experimental animal according to predetermined animal experimental groups. Each rat was sacrificed 24 hours after the blockage/reperfusion and drug injection to the left middle cerebral artery. Before and after the surgery, the rats had free access to fodder and water.

### B. Measurement of volume of cerebral infarction

2,3,5-triphenyltetrazolium chloride (TTC) dye was used to determine the area of a cerebral infarction.

First, zoletile and rompun were injected into muscles of rats to euthanize the rats, and the brains of the rats were carefully separated from their crania, which were then immersed in a 0.9% saline solution that was cooled for 1 minute. Thereafter, rodent brain matrix (RBM-4000C, ASI Instruments, Warrin, MI, USA) was used to slice the brains according to the coronal plane of the brain at 1 mm from the front pole at a thickness of 2 mm.

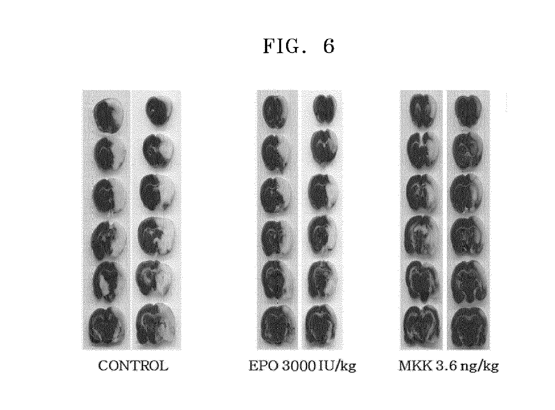

Slices obtained therefrom were stored in a phosphate-buffer (PB) including 2% 2,3,5-TTC (Sigma, St.Louis, MO, USA) at room temperature for 1 hour and then fixed with 10% phosphate-buffered formalin. Images of fixed slices of the coronal plane of the brains for different experimental groups are shown in FIG. 6.

FIG. 6 shows images showing coronal plane slices of brains which were separated from cerebral infarction induced Sprague-Dawley rat models that were fed with a test drug, and then dyed with TTC.

The cerebral infarction area was measured by an image analyzer with LeicaQwin program (Leica Microsystem Image Solution Ltd., Cambridge, England) and a volume (mm³) was calculated as a sum of infarct volumes (= infarct area of a slice x thickness) of the slices, and then an average and a standard deviation were calculated for each experimental group.

### 6. Myocardiocyte apoptosis experiment

### A. Separation and culturing of cardiomyocytes

An 8 weeks old Wistar rat was injected with ketamine and pentobarbital to quickly kill the mouse, and its heart was quickly separated from the mouse. An aorta was used to perfuse with a Ca free Krebs-Henseleit buffer (KHB) solution which was pre-heated to a temperature of about 34°C to about 37°C. Thereafter, the KHB solution was switched to Hank's balanced salt solution (HBSS:GIBCO, Grand Island, NY) containing 0.15% class II collagenase (Cooper Biomedical, Malvern, PA) and then was used to perfuse the aorta for about 30 to about 40 minutes. Thereafter, the product obtained therefrom was washed twice with a cell culture medium (Dulbecco's modified Eagle's medium, DMEM). Ventricles and atria were separated each other and only ventricular tissues were finely chopped and then stirred thoroughly for 5 minutes in a shaker filled with culture medium (DMEM) containing class II collagenase that was pre-heated to a temperature of about 34°C to about 37°C. Connective tissues that were not dissolved were filtered and a collagenase-containing solution was washed twice with the culture medium (DMEM). The product obtained therefrom was softly centrifuged at a speed of 10 g for 2 minutes, a supernatant obtained therefrom was separated and removed, and cell components were washed three times with a culture medium (DMEM) solution containing 1% fetal bovine serum (FBS; Sigma Chemical, St. Louis, MO). Thereafter, the cells obtained therefrom were stabilized in a culture medium, 10% FBS, 10mM N-2-hydroxyethylpiperazine-N'-2-ethanesulfonic acid (HEPES, GIBCO Laboratories, Grand Island, NY), 2mM L-glutamine, and penicilin-stereptomycin (100 IU/mL, GIBCO) for 60 minutes. Thereafter, the cells were divided into wells in a 6-well plate at a density of 20X10³ cells/cm² and then bromodeoxyuridine (BrdU, 10uM, Sigma-Aldrich) was added to the culture medium over 3 days to prevent the proliferation of fibroblasts and progenitor cells (Lokuta et al, 1994; Miragoli et al., 2006).
The cells were cultured in an incubator under conditions of 95% O₂, 5% CO₂, and a temperature of 37°C for the next experiment.

### B. TUNEL assay

Cultured cells were divided into a control group and an experimental group for an MKX-2 treatment, and culture media of both groups were replaced with culture media free of FBS before the MKX-2 treatment. The treatment times were 0 hour, 12 hours, and 24 hours and concentrations of MKX-2 were 0.1 ng/*µℓ* and 0.01 ng/*µℓ* for each of the control group and the experimental group. To induce apoptosis in both the control group and the experimental group, 0.03% H₂O₂ was added to both groups 30 minutes before the MKX-2 treatment.

A cover glass was placed in the 6-well plate and cells cultured thereon were washed with PBS, which were fixed with 100% ethanol for 5 seconds at room temperature and then washed again with PBS. A freshly prepared permeabilization solution (0.1% Triton X-100 + 0.1 % sodium citrate) was added to the wells and then treated for 10 minutes at a temperature of 37°C in an incubator. Thereafter, cell cultures were washed twice with PBS for 5 minutes, a mixture solution of label solution and enzyme solution in an *in situ* cell death detection kit (Roche, Germany) was dropped in an amount of 30 *µℓ* on each cover glass, which was slightly covered with a parafilm thereafter. In an incubator at a temperature of 37°C, the cell cultures were reacted for 1 hour, washed twice with PBS for 5 minutes, and then treated with DAPI (Sigma) for 10 minutes at room temperature. After washing the cell cultures with PBS, a fluorescence microscope was used to observe the cell cultures and randomly select 10 locations, count the numbers of DAPI dyed cells and TUNEL positive cells at those 10 locations, and calculate a ratio of the DAPI dyed cells to the TUNEL positive cells.

### Results

### 1. Cell protection effects of MKX-2 peptide in cardiomyocytes

Cardiomyocytes obtained from the separation and culturing of the cardiomyocytes were inoculated in a 96-well plate at a density of 2x10⁴ cells/well in an amount of 200 ul for each well. After 24 hours, when the cells had stabilized, a 26 gauge or 28 gauge needle was used to remove a preexisting medium in the wells. 200 ul of PBS free of serum and nutrients was added to each well, the wells were divided into an EPO treatment group and an MKX-2 treatment group, each of which was further divided into experimental groups according to concentrations of 0 nM, 0.1 nM, 1 nM, 10 nM, 100 nM, and 1000 nM and then treated with a drug for 72 hours, and cell survival rates of both groups were compared by using an MTT assay. The results obtained therefrom are shown in FIG. 1.

FIG. 1 is a graph showing results of comparing cell survival rates after treating cardiomyocytes with EPO and MKX-2 at various concentrations, culturing the cardiomyocytes for 72 hours, and measuring the cell survival rates through an MTT assay.

After 72 hours, when the cell survival rate was 100% for 0 nM, the cell survival rate of cells treated at a concentration of 0.1 nM was not substantially different from that of 0 nM, but the cell survival rate of cells treated at a concentration of 1 nM substantially decreased compared to the initial condition for both the EPO and MKX-2 treatment groups and there was no substantial difference between the EPO and MKX-2 treatment groups. Thereafter, at a concentration of 10 nM, the MKX-2 treatment group showed a higher cell survival rate than the EPO treatment group (102.67+3.22% vs. 94.52+4.36%, p=0.002), as well as at concentrations of 100 nM (112.28+2.17% vs. 102.64+2.69%, p<0.001) and 1000 nM (115.05+0.90% vs. 106.97+1.99%, p=0.001).

### 2. Cell protection effects of MKX-2 peptide in neurocytes

Neurocytes obtained from the separation and culturing of the neurocytes were inoculated in a 96-well plate at a density of 2x10⁴ cells/well in an amount of 200 ul for each well. After 24 hours, when the cells had stabilized, a 26 gauge or 28 gauge needle was used to remove a preexisting medium from the wells.

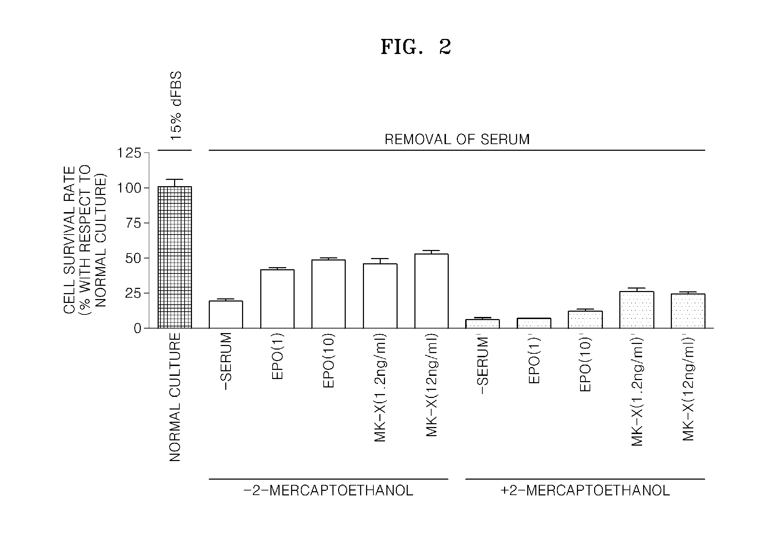

Thereafter, 200 ul of PBS free of serum and nutrients was added to the wells and the wells were divided into an EPO treatment group and an MKX-2 treatment group, wherein the EPO treatment group was further divided into a non-EPO treatment group, a 1 IU/ml treatment group, and a 10 IU/ml treatment group after being removed of serum again, each of which was then further divided into a group treated with 0.1% 2-mercaptoethanol, which is a neurotoxin, for 18 hours and a non-treatment group. Also, as a negative control group, a group cultured in a normal culture condition was used to compare the treatment group. Each group was cultured in a drug treatment environment for 72 hours and cells that survived were quantitatively analyzed by Calcein-AM assay containing a fluorescent material using a fluorometer. 1 IU/ml of EPO corresponds to 10 ng/ml, and 10 ng/ml of EPO and 1.2ng/ml of MKX-2 were used for the test, which corresponds the same concentration of 0.54 nM. Results obtained therefrom are shown in FIG. 2.

FIG. 2 is a graph showing results of comparing cell survival rate after treating brain cells with EPO and MKX-2 at various concentrations, culturing the brain cells for 72 hours, and measuring the cell survival rates through an MTT assay.

After 72 hours, when a cell survival rate of the group cultured in a normal culture condition was 100%, the EPO treatment group showed a statistically significant increase in cell survival rate that was twice as great as the cell survival rate of the non-EPO treatment group and the cell survival rate increased according to an increase in the concentration of EPO (non-treatment group: EPO(1 IU/ml): EPO(10 IU/ml)= 18.60%: 41.64%: 48.57%).

The MKX-2 treatment group also showed similar results (non-treatment group: MKX-2(1.2 ng/ml): MKX-2(12 ng/ml)= 18.60% : 46.05% : 52.85%) and there was no statistically significant difference between the EPO treatment group and the MKX-2 treatment group.

Neurocytes exposed to 2-mercaptoethanol showed a cell death rate that was about 50% higher than olfactory neurocytes that were not exposed to 2-mercaptoethanol.

After exposure to 2-mercaptoethanol, the EPO treatment group at 1U/ml did not show a statistically significant increase in cell survival rate compared to the non-treatment group (5.89% : 6.98%, p=0.19) whereas the EPO treatment group at 10 U/ml showed statistically significant neurocyte protection effects (5.89% :11.70%, p=0.02).

The MKX-2 treatment group at 1.2ng/ml showed a statistically significant increase in cell survival rate compared to the non-treatment group (5.89% : 25.72%, p=0.005), the MKX-2 treatment group at 12 ng/ml also showed neurocyte protection effects (5.89% : 24.12%, p=0.007), and the MKX-2 treatment groups at both concentrations showed higher cell survival rates than the EPO treatment groups at respective concentrations (p<0.001, P=0.002).

### 3. Cell protection effects of MKX-2 peptide in retinal neurocytes

Retinal neurocytes obtained from the separation and culturing of the retinal neurocytes were inoculated in an amount of 200 ul and at a density of 2x10⁴ cells/well for each well in a 96-well plate. After 24 hours, when the cells had stabilized, a 26 gauge or 28 gauge needle was used to remove a preexisting medium from the wells.

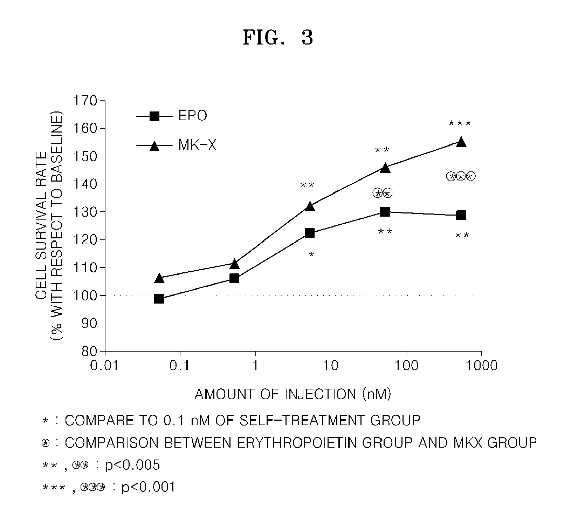

200 ul of PBS free of serum and nutrients was added to each well, the wells were divided into an EPO treatment group and an MKX-2 treatment group, each of which was further divided into experimental groups according to concentrations of 0 nM, 0.1 nM, 1 nM, 10 nM, 100 nM, and 1000 nM and then cultured for 72 hours under a drug treatment condition, and retinal neurocytes that survived were quantitatively analyzed by an MTT assay using a fluorometer. Results obtained therefrom are shown in FIG. 3.

FIG. 3 is a graph showing results of comparing cell survival rates after treating retinal neurocytes with EPO and MKX-2 at various concentrations, culturing the retinal neurocytes for 72 hours, and measuring the cell survival rates through an MTT assay.

After 72 hours, when a cell survival rate of the group cultured under a normal culture condition was 100%, cell survival rates increased for both the EPO and MKX-2 treatment groups at concentrations of 0.1 nM and 1 nM compared to 0 nM; however, without any statistical significance. Both the EPO and MKX-2 treatment groups showed a statistically significant increase in cell survival rate at 10 nM compared to 0 nM; however, there was no statistically significant difference between the EPO treatment group and the MKX-2 treatment group (128.03% vs. 118.62%, p=0.065). Both the EPO treatment group and the MKX-2 treatment group showed cell protection at 100 nM, but the MKX-2 treatment group showed a higher cell survival rate than the EPO treatment group (144.41% vs. 127.6%, p=0.002) and the MKX-2 treatment group showed a comparatively better cell survival rate than the EPO treatment group at 1000 nM (149.80% vs. 124.77%, p=0.001).

### 4. Cell protection effects of MKX-2 peptide in pancreatic β-cells

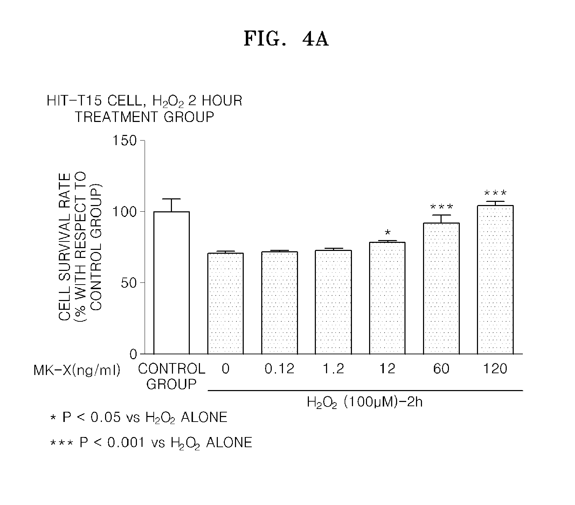

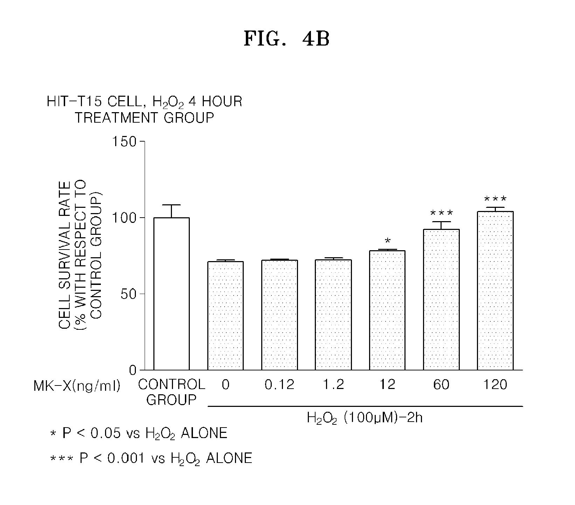

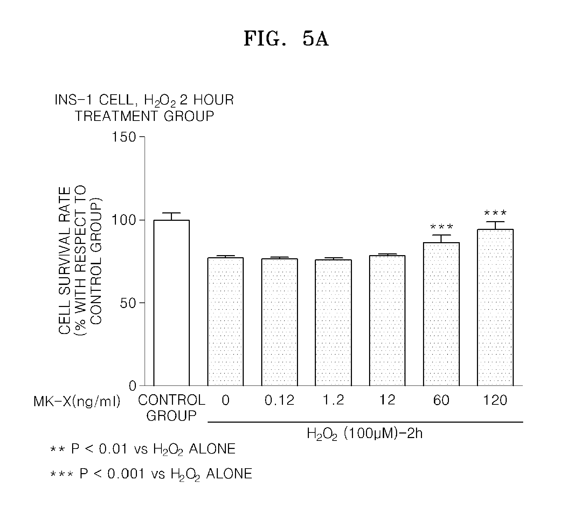

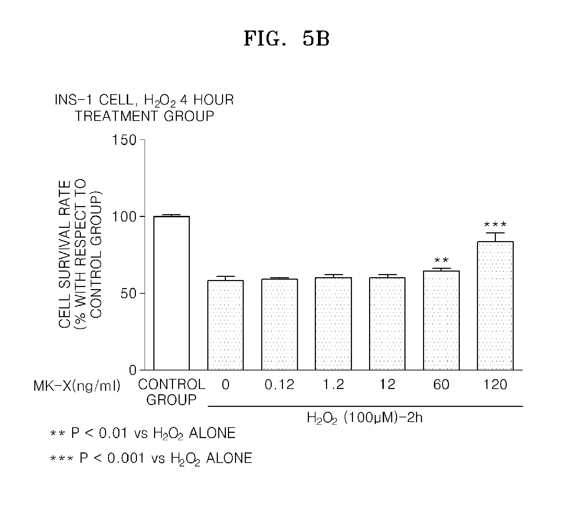

As described above, HIT-T15 and INS-1 cells, which are β-cells of islets of Langerhans, were seeded at a density of 1x10⁵ cells/well for each well in a 24-well plate, which were then stabilized, and a preexisting medium was carefully removed therefrom on the day of the experiment, including even a miniscule amount of medium that remained after the removal. Thereafter, RPMI-1640 medium including 0.5% FBS was used to culture the β-cells for 6 hours and stop proliferation and differentiation of the β-cells. An MKX-2 treatment group and a control group only including the same amount of DMSO, which was used to dissolve MKX-2, were prepared. Thereafter, the MKX-2 treatment group was treated with in amounts of 0 ng/ml, 0.12 ng/ml, 1.2 ng/ml, 12 ng/ml, 60 ng/ml, and 120 ng/ml and then the β-cells were treated with H₂O₂ (100 M) for about 2 hours to about 4 hours to put the β-cells under stress, and then an MTT assay was used to compare cell survival rates. Results obtained therefrom are shown in FIGS. 4 and 5.

FIG. 4 shows graphs showing results of comparing cell survival rates after pre-treating HIT-T15 β-cells with MKX-2 at various concentrations, treating the same with H₂O₂ for 2 hours (FIG. 4A) or 4 hours (FIG. 4B), and measuring the cell survival rates through an MTT assay.

FIG. 5 shows graphs showing results of comparing cell survival rate after pre-treating INS-1 β-cells with MKX-2 at various concentrations, treating the same with H₂O₂ for 2 hours (FIG. 5A) or 4 hours (FIG. 5B), and measuring the cell survival rates through an MTT assay.

When a cell survival rate of MKX-2 treatment at a concentration of 0 ng/ml after 2 hours or 4 hours of H₂O₂ treatment was 100%, β-cells treated with MKX-2 at concentrations of 0.12 ng/ml and 1.2 ng/ml did not show a statistically significant difference in cell survival rates compared to a non-MKX-2 treatment group. However, HIT-T15 β-cells treated with MKX-2 at concentrations of 12 ng/ml, 60 ng/ml, and 120 ng/ml showed a statistically significant increase in cell survival rates (H₂O₂ treatment for 2 hours: 78.5%+0.92(12 ng/ml), 92.3%+5.24(60 ng/ml), 104.1%+2.87(120 ng/ml) vs. 71%+1.44 (not treated); H₂O₂ treatment for 4 hours: 59.57%+2.09(12 ng/ml), 67.19%+1.05 (60 ng/ml), 84.73%+4.82 (120 ng/ml), vs. 52.46%+1.20 (not treated)), and INS-1 cells showed a statistically significant increase in cell survival rates at concentrations of 60 ng/ml and 120 ng/ml (H₂O₂ treatment for 2 hours: 86.4%+4.79 (60 ng/ml), 95.0%+4.53 (120 ng/ml), vs. 77.6%+1.02 (not-treated); H₂O₂ treatment for 4 hours: 64.39%+1.65(60 ng/ml), 83.60%+5.76(120 ng/ml), vs. 58.26%+2.56 (not treated)).

### 5. Tissue protection effects of MKX-2 in cerebral infarction animal experiment

According to the experimental method using cerebral infarction animal models described above, each experimental group was compared to a negative control group, an MKX-2 treatment group, and an EPO treatment group (positive control group).

For the negative control group, 2 hours after a middle cerebral artery was blocked, a nylon thread that induced the blockage was removed to induce a complete reperfusion and a 0.9% biological saline solution was immediately intravenoul thereto. For the MKX-2 treatment group, the same processes were repeated, except that 3.6 µg/kg of MKX-2 was injected instead of the 0.9% normal saline solution, and for the EPO treatment group, 3000 IU/kg of EPO (Eporon, Dong-A Pharmaceuticals) was injected instead of the 0.9 normal saline solution.

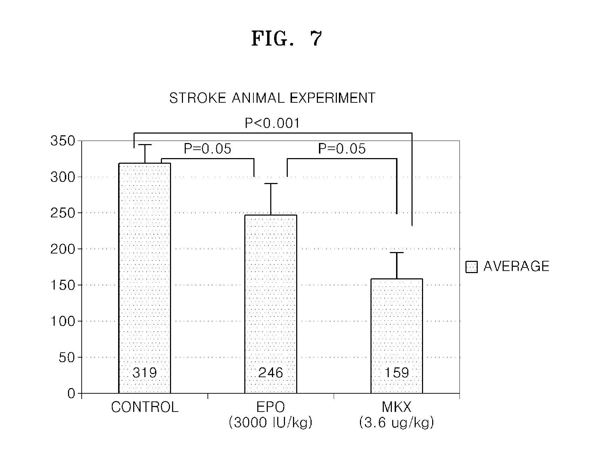

After 24 hours, experimental animals were sacrificed according to the method described above, their brains were taken out, and the volumes of cerebral infarction were measured by the method described above. Results obtained therefrom are shown in FIG. 7 as a graph.

FIG. 7 is a graph showing measurement results of a cerebral infarction area of a frontal slice of brain which was separated from a cerebral infarction induced Sprague-Dawley rat model that was fed with a test drug, and then dyed with TTC.

After 24 hours, a volume of cerebral infarction of the negative control group including a 0.9% normal saline solution was 318.70 + 29.64 mm³, the volume of cerebral infarction of the EPO treatment group was 246.27+37.52 mm³, the volume of cerebral infarction of the MKX-2 treatment group was 159.24+27.78 mm³ and thus, it may be concluded that the EPO treatment group showed a statistically significant decrease in the volume of cerebral infarctioncompared to the negative control group (318.70+29.64 mm³ vs. 246.27+37.52 mm³, p=0.05) and that the MKX-2 treatment group also showed a statistically significant decrease in the volume of cerebral infarction compared to the negative control group (318.70+29.64 mm³ vs. 159.24+27.78 mm³, p<0.001). Also, in a comparison between the EPO treatment group and the MKX-2 treatment group, the MKX-2 treatment group showed a comparatively more statistically significant decrease in the volume of cerebral infarction than the EPO treatment group (246.27+37.52 mm³ vs. 159.24+27.78 mm³, p=0.05).

### 6. Cell protection effects of MKX-2 in myocardiocyte apoptosis experiment

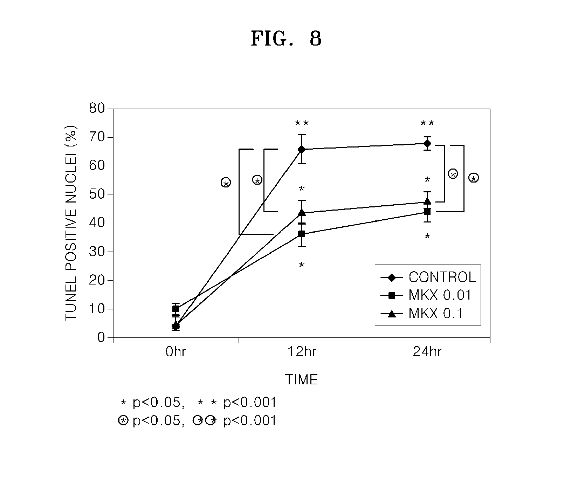

According to the myocardiocyte apoptosis experiment described above, the experimental group was divided into a control group and an MKX-2 treatment group, which were treated for 0 hour, 12 hours, and 24 hours, and each MKX-2 treatment group was divided into a 0.01 ng/uL treatment group and a 0.1 ng/uL treatment group. After the reference time, 12 hours, and 24 hours, the numbers of DAPI dyed cells and TUNEL positive cells in the control group were counted and a ratio of the DAPI dyed cells to the TUNEL positive cells was calculated. The same method was used to treat the MKX-2 treatment group at concentrations of 0.01 ng/uL and 0.1 ng/uL, and after 0 hour, 12 hours, and 24 hours, the number of DAPI dyed cells and TUNEL positive cells were counted and a ratio thereof was calculated. Results obtained therefrom are shown in FIG. 8.

FIG. 8 is a graph showing the results of counting the number of DAPI dyed cells and TUNEL positive cells with respect to cultured cardiomyocytes, which were obtained from a Wistar rat,and calcuating the ratio therefrom.

The numbers of TUNEL positive cells / the numbers of DAPI dyed cells after 0 hour, 12 hours, and 24 hours were 3.74+0.67%, 65.52+5.09%, and 68.13+2.27%, respectively, and compared to the number of cells after 0 hour, the numbers of cells after 12 hours (3.74+0.67% vs. 65.52+5.09%, p<0.001) and 24 hours (3.74+0.67% vs. 68.13+2.27%, p<0.001) showed a statistically significant increase.

The numbers of TUNEL positive cells/ the numbers of DAPI dyed cells of the MKX-2 treatment group at 0.01 ng/uL after 0 hour, 12 hours, and 24 hours were 10.12+2.03%, 35.82+3.95, 44.16+3.34%, respectively. Compared to the number of cells after 0 hour, the numbers of cells after 12 hours (10.12+2.03% vs. 35.82+3.95%, p<0.05) and 24 hours (10.12+2.03% vs. 44.16+3.34%, p<0.05) showed a statistically significant increase. The numbers of TUNEL positive cells/ the numbers of DAPI dyed cells of the MKX-2 treatment group at 0.1 ng/uL after 0 hour, 12 hours, and 24 hours were 5.38+2.36%, 44.35+3.91 %, and 47.97+2.85%, respectively. Compared to the number of cells after 0 hour, the numbers of cells after 12 hours (5.38+2.36% vs. 44.35+3.91 %, p<0.05) and 24 hours (5.38+2.36% vs. 47.97+2.85%, p<0.05) showed statistically similar results as the MKX-2 treatment group.

The numbers of TUNEL positive cells/ the numbers of DAPI dyed cells of the control group, MKX-2 treatment group at 0.01 ng/uL, and MKX-2 treatment group at 0.1 ng/uL were not statistically significant, but compared to the control group after 12 hours of the treatment, the MKX-2 treatment group at 0.01 ng/uL (65.52+5.09% vs. 35.82+3.95, p<0.05) and the MKX-2 treatment group at 0.1 ng/uL (65.52+5.09% vs. 44.35+3.91 %, p<0.05) showed a statistically significant difference. Similarly, MKX-2 treatment group at 0.01 ng/uL (68.13+2.27% vs. 44.16+3.34%, p<0.05) and MKX-2 treatment group at 0.1 ng/uL (68.13+2.27% vs. 47.97+2.85%, p<0.05) after 24 hours showed a statistically significant difference compared to the control group,. The MKX-2 treatment group at 0.01 ng/uL and the MKX-2 treatment group at 0.1 ng/uL did not show a statistically significant difference after 12 hours and 24 hours.

The results described above indicate that the MKX-2 cell protection functions through an anti-apoptosis mechanism.

## Claims

1. A peptide comprising an amino acid sequence of SEQ ID NO.1, wherein the peptide is effective for prevention or treatment of damage to cells, tissues, or organs.

2. The peptide of claim 1, wherein the peptide is derived from erythropoietin (EPO).

3. A pharmaceutical composition for prevention or treatment of damage to cells, tissues, or organs, the pharmaceutical composition comprising the peptide according to claim 1 or claim 2 as an active component.

4. The pharmaceutical composition of claim 3, wherein the cells, the tissues, or the organs are cells, tissues, or organs of a spinal cord, brain, heart, retina, skin, muscle, lung, liver, kidney, small intestine, adrenal glands, blood vessels, bladder, urethra, stomach, intestinal tract, nerves, testis, ovary, or uterus.

5. The pharmaceutical composition of claim 3, wherein the damage is caused by stroke, mechanical damage or ischemic damage to a nervous system, multiple sclerosis, seizure, low blood pressure, cardioplegia, ischemia, myocardial infarction, inflammation, age-related loss of cognitive function, radiation injury, cerebral palsy, neurodegenerative diseases, Alzheimer's disease, Parkinson's disease, Leigh's disease, AIDS dementia, memory impairment, amyotrophic lateral sclerosis, alcohol addiction, mood disorders, anxiety disorders, attention deficit disorder, autism, Creutzfeldt-Jakob disease, brain or spinal cord trauma or ischemia, heart-brain by-pass, chronic heart failure, decreased vision due to aging, diabetic nerve failure, diabetic renal failure, glaucoma, retinal ischemia, or retinal trauma.


<110>    KIM, Hoojung

<120>    Erythropoietin-derived peprides and uses thereof

<130>    px042185PCT

<160>    1

<170>    KopatentIn 2.0

<210>    1
<211>    21
<212>    PRT
<213>    Artificial Sequence

<220>
<223>    peptide sequence synthesized according to a solid-phase synthesis
         technique

<400>    1
Ile Ser Gly Leu Arg Ser Leu Thr Thr Leu Leu Arg Ala Leu Gly Ala
  1               5                  10                  15

Gln Lys Glu Leu Met
             20